Europäisches Patentamt

European Patent Office

Office européen des brevets

(11) Veröffentlichungsnummer: **0 173 915 B1**

(19)
(12)

# EUROPÄISCHE PATENTSCHRIFT

(45) Veröffentlichungstag der Patentschrift: **12.08.92**

(21) Anmeldenummer: **85110496.8**

(22) Anmeldetag: **21.08.85**

(51) Int. Cl.5: **C12N 11/04**, C12G 1/06,
//C12N11/10

Ein Antrag gemäss Regel 88 EPÜ auf
Berichtigung liegt vor. Über diesen Antrag wird
im Laufe des Verfahrens von der
Prüfungsabteilung eine Entscheidung getroffen
werden.

Die Akte enthält technische Angaben, die nach
dem Eingang der Anmeldung eingereicht wurden
und die nicht in dieser Patentschrift enthalten sind.

(54) **Biokatalysator und Verfahren zu seiner Herstellung.**

(30) Priorität: **07.09.84 DE 3432923**

(43) Veröffentlichungstag der Anmeldung:
**12.03.86 Patentblatt 86/11**

(45) Bekanntmachung des Hinweises auf die
Patenterteilung:
**12.08.92 Patentblatt 92/33**

(84) Benannte Vertragsstaaten:
**AT BE CH DE FR GB IT LI LU NL SE**

(56) Entgegenhaltungen:
**DE-A- 3 012 233**

**PATENT ABSTRACTS OF JAPAN, Band 6, Nr.
257 (C-140)[1135], 16. Dezember 1982; & JP-
A-57 150 385 (TAKARA SHUZO K.K.)
17-09-1982**

**ENCYCLOPEDIA OF FOOD SCIENCE, AVI.
1978**

(73) Patentinhaber: **Klein, Joachim,Prof. Dr.
Hühnerkamp 21
W-3300 Braunschweig(DE)**

Patentinhaber: **Vorlop, Klaus-Dieter, Prof. Dr.
Hochstrasse 7
W-3300 Braunschweig(DE)**

Patentinhaber: **Steinert, Hans-Jürgen, Dr.
Maschtor 3
W-3152 Ilsede 4(DE)**

(72) Erfinder: **Klein, Joachim,Prof. Dr.
Hühnerkamp 21
W-3300 Braunschweig(DE)**
Erfinder: **Vorlop, Klaus-Dieter, Prof. Dr.
Hochstrasse 7
W-3300 Braunschweig(DE)**
Erfinder: **Steinert, Hans-Jürgen, Dr.
Maschtor 3
W-3152 Ilsede 4(DE)**

EP 0 173 915 B1

MICROCAPSULE PROCESSING AND TECH-
NOLOGY, 1979, Seiten 59-69, Asaji Kondo:
"In-liquid curing coating process (Ortifice
Process)

Wang, H.Y. et al. (1982) Biotechnology and
Bioenginering Symp. No. 12, 139-146

74 Vertreter: **Lins, Edgar, Dipl.-Phys. et al**
**Patentanwälte Gramm + Lins Theodor-**
**Heuss-Strasse 2**
**W-3300 Braunschweig(DE)**

## Beschreibung

Die Erfindung betrifft ein Verfahren zur Herstellung eines Biokatalysators mit immobilisierten Zellen, bei dem die Zellen in eine durch ein Vernetzungsmittel zum Gel werdende, aus einer wäßrigen Phase gebildeten Flüssigkeit eingebracht werden und anschließend durch Vernetzung der Biokatalysator mit oder ohne einen mechanischen Träger gebildet wird. Die Erfindung betrifft ferner einen Biokatalysator mit immobilisierten Zellen.

Biokatalysatoren werden für die verschiedensten Anwendungsgebiete eingesetzt. Mit ihnen lassen sich beispielsweise Arzneimittel, wie Antibiotika, oder Äthanol herstellen, Abwässer reinigen o. dgl. Bekannt ist der Einsatz der entsprechenden Zellen bei der Sektherstellung, wo durch Zugabe von Hefe eine zweite Gärung in Gang gebracht wird. Diese Gärung wird in großen Tanks oder in der Sektflasche vorgenommen. Diese Verfahren gestalten sich dadurch besonders aufwendig, daß die freien, aktiven Zellen nach der Durchführung der Gärung aus der Flüssigkeit entfernt werden müssen. Bei der Flaschengärung werden die Flaschen hierzu gerüttelt und nach und nach auf den Kopf gestellt, so daß sich die Zellen im Flaschenhals absetzen. Durch Vereisung des Flaschenhalses wird mit den Zellen ein entfernbarer Pfropfen gebildet. Bei der Tankgärung muß eine Filtration des Sektes vorgenommen werden.

Es ist seit einiger Zeit bekannt, für derartige Anwendungsfälle für Biokatalysatoren immobilisierte Zellen zu verwenden. Diese Zellen sind durch geeignete Maßnahmen an einen Ort gebunden, bleiben aber weiterhin lebensfähig und aktiv. Nach der Durchführung der mikrobiologischen Reaktion können sie zusammen mit der Immobilisierungsmasse im allgemeinen leicht entfernt werden. Es ist bekannt, eine derartige Immobilisierung durch in wäßriger Phase vernetzende Gele zu bewerkstelligen (vgl. Klein, Wagner "Methods for the Immobilization of Microbial Cells" in Applied Biochemistry and Bioengineering 1983 Vol. 4, Seiten 11 - 51).

In der Praxis hat es sich jedoch gezeigt, daß für längerwährende Vorgänge, wie beispielsweise die Sektgärung, die durch die Immobilisierung angestrebten Effekte nicht erzielt werden. Es zeigt sich nämlich, daß die Zellen aus dem Immobilisierungsmaterial "auswachsen". Zwar verhindern die Gele durch ihre Netzstruktur ein Auswandern der eingeschlossenen Zellen, es läßt sich aber nicht vermeiden, daß bei der Herstellung der Gele auch Zellen an der Oberfläche des Biokatalysators sitzen. Diese finden gegenüber den eingeschlossenen Zellen begünstigte Ernährungsbedingungen vor und vermehren sich wesentlich stärker als die eingeschlossenen Zellen. Diese Zellen nehmen als freie Zellen am Gärungsvorgang teil und gelangen in die Flüssigkeit und führen zu einer erheblichen Trübung.

Ein unter der Bezeichnung "Mikrokapselung" bekanntes Verfahren eignet sich zur Immobilisierung der Zellen nicht, weil hierbei ein Polymer in einem organischen Lösungsmittel gelöst werden muß, das die Zellen praktisch vollständig abtötet. Die verbleibende Aktivität des Katalysators würde in den meisten Fällen nicht ausreichen. Darüber hinaus läßt sich auch bei der Mikrokapselung ein Auswachsen der Zellen nicht sicher verhindern.

In der Veröffentlichung "Biotechnology and Bioengineering" Symp. No. 12, Seiten 139 - 146 (1982) ist untersucht worden, in welcher Menge Zellen aus einem Biokatalysator herausgelangen können. Die Biokatalysatoren sind dabei in einer Ringer-Salzlösung gerührt worden. Da die Salzlösung keine Nährlösung für die Zellen ist, beruht der Übertritt von Zellen aus dem Biokatalysator in die Salzlösung auf rein mechanischen Effekten, also nicht auf einem "Auswachsen" der Zellen aus dem Biokatalysator. Von den Autoren ist vorgeschlagen worden, den Übertritt von Zellen aus dem Biokatalysator in die umgebende Lösung dadurch zu vermindern, daß die Katalysatorperlen mit den immobilisierten Zellen mit einer Schutzschicht überzogen werden, beispielsweise aus Ca-Alginat. Angaben zur Herstellung dieser Beschichtung sind in der Veröffentlichung nicht enthalten.

Durch die unter der Nummer 57-150 385 veröffentlichte japanische Patentanmeldung ist ein Verfahren zur Herstellung einer derartigen Schutzschicht um eine Perle mit immobilisierten Zellen offenbart. Danach wird die Perle beispielsweise in eine Natrium-Alginatlösung getaucht. Die so mit der Alginatlösung benetzten Perlen werden ausgehoben und anschließend in eine Calcium-Salzlösung und gegebenenfalls anschließend sofort in eine Aluminium-Salzlösung getaucht. Dabei wird zunächst das Gel zu Calcium-Alginat vernetzt und anschließend Aluminium-Alginat gebildet, wobei ein Austausch von Calcium-Ionen durch Aluminium-Ionen stattfindet. Diese zweistufige Bildung der Gelschicht wird durchgeführt, um zunächst zu einer Vernetzung zu einer Schutzschicht bei einem für die Biomasse unkritischen pH-Wert zu gelangen. Das die Biomasse in der Perle gefährdende saure Aluminiumsalz wird durch die gebildete Ca-Schutzschicht von der Biomasse abgehalten, so daß sich nun eine mechanisch stabilere Aluminium-Alginatschicht bilden kann, ohne daß die Biomasse ihre Aktivität verliert.

Durch die FR-A 2 452 285 (= DE-A 30 12 233) ist es ferner bekannt, eine in herkömmlicher Weise gebildete Perle, beispielsweise aus Calcium-Alginat, mit immobilisierten Zellen mit einer semipermeablen Membran zu umhüllen. Hierzu wird die Oberfläche der gebildeten Perlen mit einem Polymerisat zusätzlich vernetzt, das mit Funktionsgruppen der gebildeten Perle Bindungen eingeht. Geeignet hierfür sind Polymerisate mit Amin- oder Imingruppen, z. B. Polyäthylen-Imin oder Polylysin, wobei die Dicke der gebildeten Membran von der durch das Molekulargewicht des Polymerisats bestimmten Eindringtiefe in die Perle abhängt. In einem alternativen Verfahren werden die Perlen mit einem Polymerisationspartner in einer wäßrigen Lösung suspensiert und mit dieser wäßrigen Suspension eine Wasser-in-Öl-Emulsion hergestellt, in deren hydrophobe Phase der andere Polymerisationspartner gelöst ist. An der Tröpfchengrenze der Emulsion findet die Polymerisationsreaktion zur Bildung der extrem dünnen semipermeablen Membran statt, die somit nicht an der Oberfläche der Perle entsteht.

Es hat sich gezeigt, daß die bekannten Verfahren nicht das Auswachsen von Zellen aus dem Biokatalysator verhindern, so daß zumindest bei langzeitigen Anwendungen freie Zellen in die den Biokatalysator umgebenen Flüssigkeit gelangen.

Der Grund für das weiterhin beobachtete Auswachsen besteht darin, daß bei der Bildung der äußeren Schutzschicht die an der Oberfläche der Perle sitzenden Zellen in die Schutzschicht hineinwandern können und daher - zumindest nach einer gewissen Zeit - nicht mehr am Auswachsen gehindert werden können.

Der Erfindung liegt die Aufgabe zugrunde, einen Biokatalysator zu erstellen, bei dem ein Auswachsen der Zellen auch bei Langzeitverfahren sicher verhindert wird.

Diese Aufgabe wird erfindungsgemäß bei einem Verfahren der eingangs erwähnten Art dadurch gelöst, daß der die Zellen enthaltende Kern von einer gelbildenden Flüssigkeit umgeben wird und daß die Vernetzung des Gels unter Einwirkung eines aus dem Kern diffundierenden Vernetzungsmittels an dem Kern erfolgt, so daß eine von den Zellen freie Schutzschicht aus dem vernetzten, keine Durchlässigkeit für die Zellen aufweisenden Gel gebildet wird, die den die Zellen enthaltenden Kern umschließt.

Demgemäß wird die zusätzliche Schicht aus einem vernetzten Gel so gebildet, daß eine Vernetzung vom Kern ausgeht, also von innen nach außen fortschreitet. Dies geschieht dadurch, daß der Kern ein Vernetzungsmittel für das die äußere Schicht bildende, vorzugsweise ionotrope Gel enthält. Dies hat zur Folge, daß sofort beim Aufbringen der gelbildenden Flüssigkeit auf den zellenhaltigen Kern die Vernetzung beginnt, so daß extrem schnell an der zum Kern zeigenden inneren Oberfläche der äußeren Schicht eine für die Zellen undurchlässige vernetzte Struktur gebildet wird. Dadurch wird das Einwandern der Zellen aus dem Kern in die umgebende Schutzschicht verhindert, so daß die Schutzschicht frei von Zellen bleibt.

Bei dem bekannten Verfahren, die umgebende Schutzschicht von außen nach innen zu vernetzen, indem das Vernetzungsmittel bzw. der Polymerisationspartner der vernetzenden Flüssigkeit von außen zugeführt wird (vgl. JP-A 57-150 385) bildet sich die erste Vernetzungsschicht an der äußeren Oberfläche und muß nach innen fortschreiten. Solange die inneren Bereiche der Schutzschicht noch nicht vernetzt sind, können Zellen in die Schutzschicht einwandern. Darüber hinaus bildet die von außen nach innen vernetzte Schutzschicht eine Porenstruktur, dessen Porengröße nach innen hin zunimmt, so daß die Zellen leicht durch die großen Poren in die Schutzschicht eindringen können.

Die äußere Schutzschicht der erfindungsgemäßen Biokatalysatoren weist hingegen unmittelbar am Kern die kleinste Porengröße auf, da die Vernetzung von innen nach außen fortschreitet. Selbstverständlich ist es möglich und vorteilhaft, zusätzlich das Vernetzungsmittel auch von außen zuzuführen, so daß eine stabile, kleinporige vernetzte Schicht sowohl an der inneren als auch an der äußeren Oberfläche der den Kern umgebenden Schutzschicht entsteht.

Der die Zellen enthaltende Kern kann in herkömmlicher Weise gebildet sein, also bereits in einem Gel immobilisierte Zellen aufweisen. Die Bildung geschieht dabei vorzugsweise durch Einbringen der mit den Zellen versehenen gelbildenden Flüssigkeit in ein Vernetzungsmittel, wodurch beispielsweise Perlen o. ä. gebildet werden. Unter Ausnutzung des in dem Kern noch befindlichen Vernetzungsmittels wird die Anordnung nunmehr vorzugsweise in dieselbe gelbildende Flüssigkeit getaucht, wie sie für den Kern verwendet worden ist. Unter Einwirkung des aus dem Kern diffundierenden Vernetzungsmittels entsteht an dem Kern die dünne Schutzschicht. Die Stärke der Schutzschicht kann durch die Konzentration der gelbildenden Flüssigkeit und die Reaktionszeit gesteuert werden. Vorzugsweise liegt diese niedriger als bei der gelbildenden Flüssigkeit des Kerns. Nach Ausbildung der Schutzschicht wird der Biokatalysator wiederum in das Vernetzungsmittel getaucht, wodurch die Schutzschicht stabil vernetzt.

Die obenerwähnte Aufgabe wird ferner bei einem Verfahren der eingangs erwähnten Art dadurch gelöst, daß die die Zellen enthaltende Flüssigkeit von der gelbildenden Flüssigkeit umgeben wird und anschließend vor einer Vermischung der die Zellen enthaltenden Flüssigkeit mit der gelbildenden Flüssigkeit in das Vernetzungsmittel gelangt, wodurch eine keine Durchlässigkeit für die Zellen aufweisende Schutzschicht durch das vernetzte Gel gebildet wird.

Bei diesem einstufigen Verfahren wird der die Zellen enthaltende Kern vor dem Vernetzungsvorgang mit der die Schutzschicht bildenden gelbildenden Flüssigkeit umgeben. Hierbei kann der Kern weiterhin flüssig sein. Die aktiven Substanzen werden dabei in die die Schutzschicht bildende gelbildende Flüssigkeit eingebracht, ohne sich mit ihr zu vermischen. Dies kann durch Einstellung unterschiedlicher Viskositäten der Kernflüssigkeit und der gelbildenden Flüssigkeit geschehen. Daraufhin kann die gesamte Anordnung in ein Vernetzungsbad getaucht werden, das ein Vernetzungsmittel enthält.

Für die Durchführung des Verfahrens eignet sich besonders eine Düsenanordnung mit einer zentralen Austrittsöffnung für die Substanz des Kerns und eine darum angeordnete ringförmige Austrittsöffnung für die gelbildende Flüssigkeit der Schutzschicht.

Das Eintauchen muß sofort nach dem Umgeben des Kerns mit der zellenfreien Flüssigkeit erfolgen. Dann durchdringt das Vernetzungsmittel die Schutzschicht so schnell, daß die Immobilisierung der Zellen im Kern so rechtzeitig erfolgt, daß keine Zellen in die Schutzschicht eindringen können. Bei diesem Verfahren ist es wesentlich, daß sich die Kernflüssigkeit und die gelbildende Flüssigkeit für die Schutzschicht nicht auf dem Wege in das Vernetzungsbad vermischen. Durch entsprechende Zugaben in die Kernflüssigkeit kann dafür gesorgt werden, daß beide Flüssigkeiten eine stark unterschiedliche Viskosität und Oberflächenspannung aufweisen, so daß die Durchmischung gehemmt wird. Hierzu kann der Kernflüssigkeit beispielsweise ein Verdickungsmittel, das nicht aushärtend sein muß, zugegeben werden.

Eine besonders stabile Schutzschicht wird auch im einstufigen Verfahren erzielt, wenn die Substanz des Kerns auch ein Vernetzungsmittel für die gelbildende Flüssigkeit der Schutzschicht enthält. Beim Einbringen der Gesamtanordnung in das Vernetzungsmittel findet die Vernetzung der Schutzschicht sowohl von innen als auch von außen statt, wobei auch hierbei der Kern flüssig bleiben kann.

Die erfindungsgemäßen Verfahren bieten den weiteren Vorteil, daß die Züchtung von Zellen bereits in deren immobilisierten Zustand erfolgen kann. Es ist nunmehr möglich, in dem Kern des Biokatalysators nur eine Vorkultur der Zellen einzuschließen und den Biokatalysator in eine für die Züchtung der Zellen geeignete Umgebung einzubringen, nämlich in eine Nährlösung, die auf einer geeigneten Temperatur gehalten wird und gegebenenfalls beispielsweise mit Sauerstoff begast wird. Die Züchtung der Zellen im immobilisierten Zustand war bisher nicht möglich, weil die Züchtung wegen des Auswachsens der Zellen im wesentlichen an der Oberfläche stattgefunden hätte und daher überwiegend freie Zellen produziert worden wären. Da bei dem erfindungsgemäßen Verfahren eine von den Zellen freie Schutzschicht vorhanden ist, die das Auswachsen sicher verhindert, kann der Biokatalysator mit der Vorkultur in die Nährlösung eingebracht werden, ohne daß die Produktion von freien Zellen an der Oberfläche des Biokatalysators zu befürchten wäre. Dadurch ist es möglich, das aufwendige Zentrifugieren der gezüchteten Zellen aus der Nährlösung zu vermeiden, da die Zellen bereits im immobilisierten Zustand gezüchtet werden können.

Die obenerwähnte Aufgabe wird weiterhin gelöst durch einen Biokatalysator mit immobilisierten Zellen, bei dem die Zellen in eine durch eine wäßrige Phase gebildete Flüssigkeit eingebracht sind und dessen Oberfläche durch eine Schutzschicht aus einem vernetzten, den die Zellen enthaltenden Kern mit oder ohne einen mechanischen Träger umschließenden Gel gebildet ist, wenn die Schutzschicht frei von den Zellen und mit einem aus dem Kern diffundierenden Vernetzungsmittel gebildet ist.

Das Auswachsen der Zellen, das beispielsweise beschrieben ist in Stöcklein, Eisgruber, Schmidt "Conversion of L-Phenylalanine to L-Tyrosine by Immobilized Bacteria" in Biotechnology Letters Vol. 5 No. 10, Seiten 703 - 708 (1983), wird erfindungsgemäß mit einem geringen zusätzlichen Aufwand vermieden. Der bei der Sektherstellung für die Trennung der freien Zellen von der Sektflüssigkeit bisher erforderliche Aufwand entfällt völlig.

Als mit einem Vernetzungsmittel vernetzenden Gele kommen beispielsweise die ionisch vernetzenden Gele Ca-Alginat, K- und/oder Ca-Kappa-Carrageenan, Chitosan, Mg- oder Ca-Pektinat, Carboxymethylcellulose oder die kovalent vernetzenden Gele Polyacrylamid, Polyurethan, Polyepoxid, Silicagele o. a. in Frage. Für die Sektherstellung ist Ca-Alginat besonders geeignet, wobei als gelbildende Flüssigkeit Na-Alginat und als Vernetzungsmittel $CaCl_2$ verwendet werden, die beide im Lebensmittelbereich seit vielen Jahren verwendet werden und daher unbedenklich sind.

Die Schutzschicht kann auch aus mehreren Komponenten gebildet sein. So kann es vorteilhaft sein, daß die Schutzschicht aus einem ionotropen Gel gebildet ist, das eine zur kovalenten Vernetzung befähigte Substanz enthält. Solche Substanzen sind beispielsweise Isocyanat-, Epoxy- und Silicon-Präpolymere.

Beispiel 1

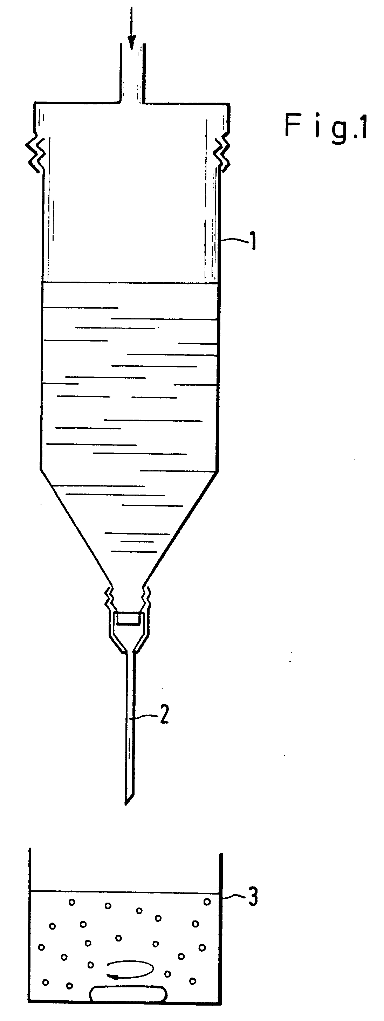

Zur Herstellung eines Biokatalysators in Form von Perlen wird eine 3,3 %ige Na-Alginatlösung durch Einrühren von 3,3 g Na-Alginat in 100 ml dest. Wasser eingerührt und anschließend 15 Minuten bei 1 bar Überdruck und bei 121 °C autoylaviert. Hefezellen (Saccharomyces bayanus; Lalvin C1108 der Boehringer KG) wird in einer 0,9 %igen NaCl-Lösung suspendiert. Die 3,3 %ige Na-Alginatlösung wird nun in der Suspension verrührt. Mit Hilfe einer in Figur 1 dargestellten Tropfarmatur 1 wird mit Hilfe von in die Armatur eingegebener Druckluft die Alginat-Zellsuspension durch eine am unteren Ende der Armatur 1 angesetzten Kanüle 2 in ein Fällungsbad getropft, das aus einer 2 %igen $CaCl_2$-Lösung besteht. Beim Eintauchen des Tropfens in das Fällungsbad entsteht eine ca. 3 mm große Gelperle, da durch Austausch der $Na^+$-Ionen durch $Ca^{2+}$-Ionen die Vernetzungs des Alginats zum Gel entsteht.

Die durch die ionotrope Gelbildung entstandenen Perlen werden zur weiteren Vernetzung 30 Minuten in der $CaCl_2$-Lösung gerührt. Anschließend werden die Perlen abgesiebt und mit Leitungswasser mehrmals gewaschen. Die Perlen werden dann mit dest. Wasser in eine 0,5 %ige bzw. 0,2 %ige Na-Alginatlösung gespült. Die Herstellung dieser Alginatlösung erfolgt analog wie oben beschrieben. Durch Diffusion von Ca-Ionen aus den Gelperlen in die Na-Alginatlösung bildet sich eine Hülle aus Ca-Alginat um die Perlen. Nach ca. fünfminütigem Rühren mit hängendem Rührer hat sich eine genügend dicke Hülle gebildet, so daß die Na-Alginatlösung abgegossen werden kann. Die Perlen werden mit Leitungswasser gewaschen und in einer 2 %ige $CaCl_2$-Lösung gespült. Nach einer Vernetzungszeit von einer Stunde werden die Perlen mehrfach mit Leitungswasser gewaschen.

Zur Durchführung einer Flaschengärung werden die Perlen in einem am oberen Ende als Korken 5 ausgebildeten Hohlzylinder 6 eingefüllt, dessen Boden 7 mittels eines Gewindes am Zylinder 6 befestigbar ist.

Für die Sektherstellung aus einem Rieslingwein mit 22 g Zucker/Liter und 2,6 g Weinsäure/Liter werden 0,2 g Trockehefe (Lavin C 1108) in 1 - 2 ml 0,9 %ige NaCl-Lösung suspendiert. Diese Suspension wird in 10 g der 3,3 %igen Na-Alginatlösung gegeben. Nach der Vernetzung in der 2 %igen $CaCl_2$-Lösung beträgt die Zellbeladung ca. 2 % (0,02 g Trockenhefe/g feuchtem Katalysator). Von diesem Katalysator wird jeweils soviel eingesetzt, daß 0,2 g Trockenhefe auf 1 l Cuvée kommen.

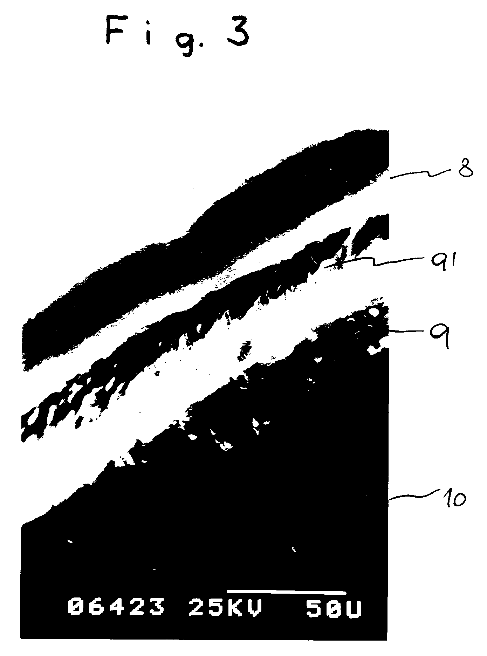

Die gebildeten Perlen werden vorsorglich 5 Minuten mit einer 6 Watt UV-Lampe bestrahlt, um etwaige, durch Verunreinigung auf die Alginathülle gelangte Zellen abzutöten. Das in Figur 3 gezeigte Bild des Katalysators zeigt eine deutliche Ca-Alginatschutzschicht 8, die frei von Zellen 9 ist, die im Kernbereich 10 deutlich zu erkennen sind. Da in dieser Abbildung die Schutzschicht zur Verdeutlichung etwas stufenartig nach hinten versetzt ist, sind die an der Oberfläche des Kerns befindlichen Zellen 9' erkennbar, die bei der herkömmlichen Immobilisierung ausgewachsen wären.

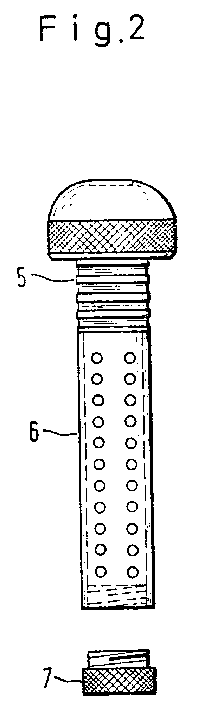

In Testansätzen in 0,7 l-Flaschen mit dem in Figur 2 dargestellten Korken bei einer Gärtemperatur von 15 °C konnte nach 4 Monaten kein Auswachsen der Hefezellen festgestellt werden. Ebenso zeigte sich bei dem Weinsäuregehalt von 2,6 g Weinsäure/l und der Verwendung eines Alginats mit einem hohen K-Wert für $Ca^{2+}$ $Na^+$ keine Ca-Tartratausscheidung.

Die Verwendung des hohlen, seitlich durchbohrten Korkens 5,6 zur Aufnahme der Perlen ermöglicht ein müheloses Entfernen der Katalysatorperlen aus den Flaschen nach Abschluß der Zweitgärung innerhalb von weniger als 2 Sekunden.

Beispiel 2

Wie beim Beispiel 1 wird eine 3,3 %ige Na-Alginatlösung angesetzt. Eine zweite, 1 %ige Na-Alginatlösung wird in gleicher Weise hergestellt. Beide Lösungen werden 15 Minuten unter den in Beispiel 1 angegebenen Bedingungen autoklaviert. Die in Beispiel 1 erwähnte Trockenhefe wird in 0,9 %iger NaCl-Lösung suspendiert und in die 3,3 %ige Na-Alginatlösung gegeben. Die Alginat-Zellsuspension wird so eingestellt, daß sich 1 g Trockenhefe in 10 g Na-Alginatlösung befinden.

Zur Herstellung eines Biokatalysators wird ein Baumwollfaden mit ca. 18 cm Länge, der alle 1,5 cm mit einem Knoten versehen ist, ausgekocht, um störende Geschmacksstoffe aus dem Baumwollfaden zu entfernen. Anschließend wird der Faden in Leitungswasser gewaschen und als Träger für das Katalysatorgel eingesetzt. In die Alginat-Zellsuspension wird der Baumwollfaden getaucht, so daß der Faden gut getränkt ist. Die Knoten sollen die Haftung des Alginatgels erleichtern. Der Baumwollfaden wird nun mit der daran haftenden AlginatZellsuspension in eine 2 %ige $CaCl_2$-Lösung zur ionotropen Gelbildung gegeben. Der Katalysatorfaden wird unter leichtem Rühren 30 Minuten in der $CaCl_2$-Lösung belassen. Anschließend wird der Fadem mit Leitungswasser gewaschen und in die 1 %ige Na-Alginatlösung getaucht. Durch Diffusion von Ca-Ionen aus dem Gel am Faden in die Na-Alginatlösung bildet sich nun eine Hülle aus Ca-Alginat um den Katalysatorfaden. Es ist darauf zu achten, daß der Katalysator am Faden ganz von der Ca-Alginathülle überzogen wird. Der Katalysatorfaden wird zur weiteren Vernetzung der Alginathülle für eine Stunde in eine leicht gerührte 2 %ige $CaCl_2$-Lösung gegeben. Anschließend wird der Katalysatorfaden mehrfach mit Leitungswasser gewaschen und dabei 5 Minuten mit einer 6 Watt UV-Lampe bestrahlt.

An dem Katalysatorfaden befindet sich in dem Alginatgel ca. 0.07 g Trockenhefe. Es werden zwei Katalysatorfäden pro 0,7 l-Flasche eingesetzt, so daß ca. 0,14 g Trockenhefe auf 0,7 l Cuvée kommen.

Die beiden Baumwollfäden werden zusammengebunden und an einem dünnen Zwirnsfaden befestigt. Der Zwirnsfaden wird aus der Flasche herausgeführt und mit einem handelsüblichen Sektkorken beim Verschließen der Flasche eingeklemmt. Auf diese leise lassen sich die Katalysatorfäden nach der Zweitgärung aus der Flasche beim Öffnen entfernen.

Während der in Figur 2 dargestellte Korken, der vorzugsweise aus Polyäthylen hergestellt ist, wiederverwendbar ist, gilt dies für den beschichteten Baumwollfaden nicht.

### Beispiel 3

Wie beim Beispiel 1 wird eine 3,3 %ige Na-Alginatlösung angesetzt. Ferner wird eine 1 %ige wässrige Na-Kappa-Carrageenanlösung in gleicher Weise hergestellt. Beide Lösungen werden 15 Minuten unter den in Beispiel 1 angegebenen Bedingungen autoklaviert. Die in Beispiel 1 erwähnte Trockenhefe wird in einer 0,9 %igen NaCl-Lösung suspendiert und in die 3,3 %ige Na-Alginatlösung gegeben. Die Alginat-Zellsuspension wird so eingestellt, daß sich 1 g Trockenhefe in 10 g Na-Alginatlösung befindet.

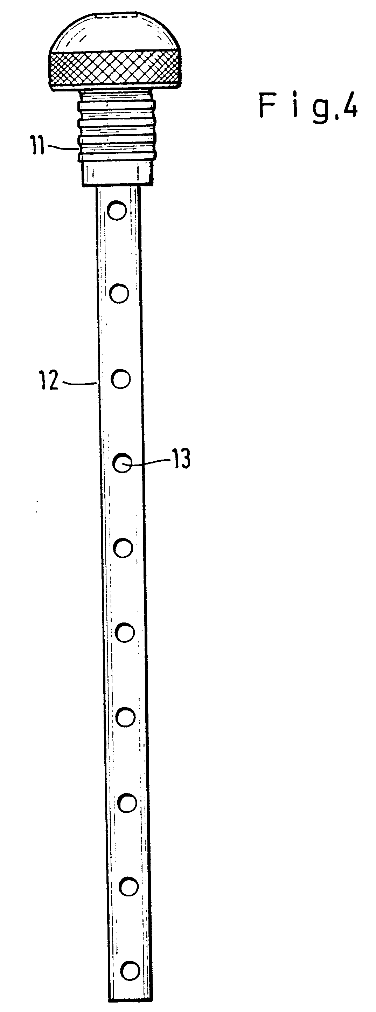

Zur Herstellung des Biokatalysators wird als Träger ein in Figur 4 dargestellter Polyäthylenkorken 11 verwendet, an dem eine flache Stange 12 befestigt ist, die in regelmäßigen Abständen Löcher 13 aufweist, die eine analoge Funktion wie die Knoten in dem in Beispiel 2 erwähnten Baumwollfaden haben. Zur Unterstützung der Haftung ist die Oberfläche der Stange 12 aufgerauht. Die Stange wird in die Alginat-Zellsuspension getaucht, so daß sie von einem Film der Alginat-Zellsuspension überzogen wird. Die überzogene Stange wird nun in eine 2 %ige $CaCl_2$-Lösung zur ionotropen Gelbildung gegeben und für 30 Minuten in der $CaCl_2$-Lösung belassen. Anschließend wird die Stange 12 gewaschen und in die 1 %ige Na-Carrageenanlösung getaucht. Durch Diffusion von Ca-Ionen aus dem Gel an der Stange 12 in die Na-Carrageenanlösung bildet sich nun eine Hülle aus Ca-Carrageenan. Der Korkenkatalysator 11,12 wird zur weiteren Vernetzung der Carrageenanhülle für eine Stunde in eine leicht gerührte Lösung bestehend aus 10 g KCl + 10 g $CaCl_2$ /1 l Wasser gegeben. Anschließend wird der Katalysator gewaschen und dabei 5 Minuten mit einer 6 Watt UV-Lampe bestrahlt.

An dem Korkenkatalysator befinden sich ca. 0,14 g Trockenhefe. Dieser Korkenkatalysator wird nun wie in Beispiel 1 beschrieben zur Sektherstellung eingesetzt. Nach abgeschlossener Zweitgärung kann der Korkenkatalysator innerhalb von 2 Sekunden aus der Flasche entfernt werden. Nach Abtrennung der Gelschichten von der Stange 12 kann der Korken 11 mit der Stange 12 erneut verwendet werden.

### Beispiel 4

Wie beim Beispiel 1 wird eine 3,3 %ige Na-Alginatlösung angesetzt und 15 Minuten unter den in Beispiel 1 angegebenen Bedingungen autoklaviert. Die in Beispiel 1 erwähnte Trockenhefe wird in 0,5 %iger $CaCl_2$-Lösung suspendiert. Die Zellsuspension wird so eingestellt, daß sich 1 g Trockenhefe in 10 g Zellsuspension befindet.

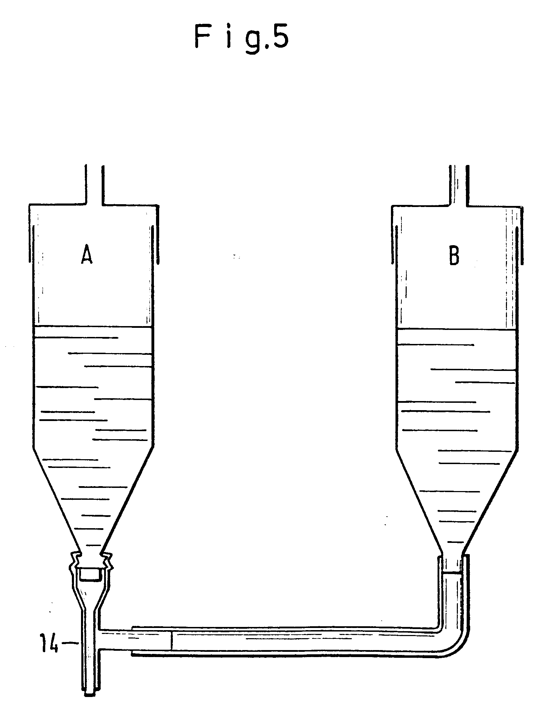

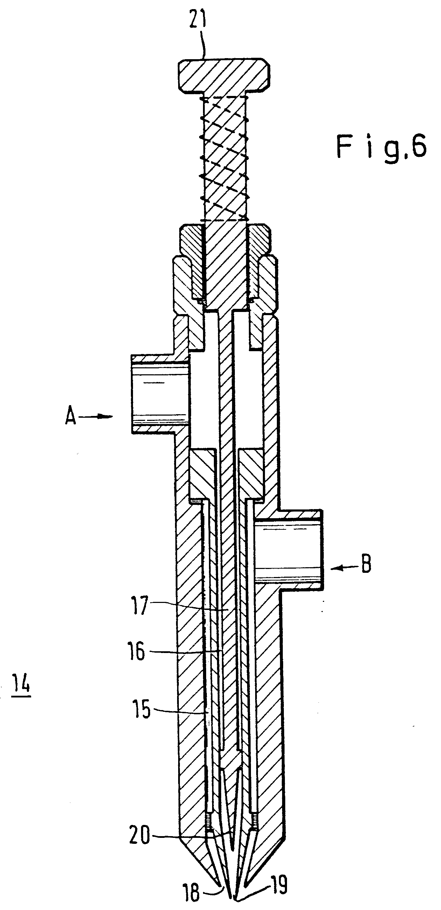

Die Katalysatorherstellung erfolgt mit der in Figur 5 und 6 dargestellten Apparatur, die aus zwei Vorratsbehältern A,B besteht, deren Inhalt in eine Zweistoffdüse 14 gelangt, deren Aufbau in Figur 6 verdeutlicht ist.

Die Zellsuspension wird in den Vorratsbehälter A und die Alginatlösung in den Vorratsbehälter B gegeben. Beide Lösungen werden nun mit einer Durchflußgeschwindigkeit von 300 ml/m durch die Zweistoffdüse 14 in eine 2 %ige $CaCl_2$-Lösung gefördert. Durch Vernetzung der Na-Alginatlösung von innen und von außen bildet sich momentan ein sehr stabiler Biokatalysatorfaden aus. Die Zweistoffdüse enthält einen äußeren ringförmigen Kanal 15 für die Lösung in dem Behältez B sowie einen inneren ringförmigen Kanal 16 für die Lösung aus dem Behälter A. Der innere ringförmige Kanal 16 wird innen durch einen verschiebbaren Kolben 17 begrenzt. Der äußere ringförmige Kanal endet in einer ringförmigen Düsenöffnung 18, die um eine zentrale Düsenöffnung 19 angeordnet ist, durch die Flüssigkeit aus dem inneren ringförmigen Kanal 16 austritt. Der Kolben 17 weist an seiner Unterseite eine Spitze 20 auf, die beim Herabdrücken des Kolbens 17 an einem Betätigungsglied 21 die Düsenöffnung 19 verschließt. Das Verschließen der Düsenöffnung 19 wird am Fadenanfang und -ende vorgenommen, um auch am Fadenanfang und am Fadenende einen dichten Abschluß mit einer zellenfreien Alginatlösung zu erhalten.

Der erhaltene Biokatalysatorfaden wird für weitere 30 Minuten in der $CaCl_2$-Lösung belassen. Anschließend wird der Katalysator mit Leitungswasser gewaschen und dabei 5 Minuten mit einer 6 Watt UV-Lampe bestrahlt. Dieser Katalysatorfaden wird nun wie in Beispiel 1 beschrieben zur Sektherstellung eingesetzt. In diesem Fall erfolgt jedoch die Sektherstellung nach dem Tankgärverfahren im Rührkessel. Hierzu werden Biokatalysatorfäden, die 0,4 g Trockenhefe/l Cuvée enthalten, eingesetzt. Nach 14 Tagen ist die Zweitgärung abgeschlossen, und der Sekt wird aus dem Gärtank abgepumpt, wobei die Katalysatorfäden durch ein Sieb mit einer Maschenweite von 2 mm zurückgehalten werden.

### Beispiel 5

Eine 1,5 %ige Alginatlösung, die analog der früheren Beispiele hergestellt worden ist, wird mit 10 g Zellen/100 g Alginatlösung versetzt. Die Zellen wurden zuvor in 0,9 %iger NaCl-Lösung suspendiert. Diese Suspension wird durch den inneren Kanal 16 der Zweistoffdüse 14 aus dem Behälter A mit Überdruck gedrückt. Eine zellfreie 3,5 %ige Alginatlösung wird durch den äußeren Ringkanal 15 aus dem Behälter B gedrückt. Für den inneren Kanal 16 ist eine Flußrate von 200 ml/h und für den äußeren Kanal 15 eine Flußrate von 300 ml/h eingestellt.

Die entstandene Kugel tropft in eine gerührte Vernetzerlösung (2 %ige $CaCl_2$-Lösung). Nach 45 Minuten Vernetzungszeit werden die Katalysatorperlen gewaschen und 5 Minuten mit einer 6 Watt UV-Lampe bestrahlt. Die nach diesem einstufigen Verfahren hergestellten Katalysatorperlen werden in gleicher Weise angewendet wie die zweistufig hergestellten Katalysatorperlen gemäß Beispiel 1.

### Beispiel 6

Eine 2%ige wässrige Na-Kappa-Carrageenanlösung wird 15 Minuten unter den in Beispiel 1 angegebenen Bedingungen autoklaviert. Die in Beispiel 1 erwähnte Trockenhefe wird in einer 1%igen KCl-Lösung suspendiert. Die Zellsuspension wird so eingestellt, daß sich 1 g Trockenhefe in 10 g Lösung befindet.

Die Katalysatorherstellung erfolgt mit der in Figur 5 und 6 dargestellten Apparatur. Die Zellsuspension wird durch den inneren Kanal 16 der Zweistoffdüse 14 aus dem Behälter A mit Überdruck gedrückt. Die zellfreie 2%ige Na-Kappa-Carrageenanlösung wird durch den äußeren Ringkanal 15 aus dem Behälter gedrückt. Für den inneren Kanal 16 ist eine Flußrate von 100 ml/min und für den äußeren Kanal 15 eine Flußrate von 500 ml/min eingestellt.

Der Faden wird bei Raumtemperatur (20°C) in eine 3%ige KCL-Lösung eingedüst. Durch Vernetzung der Carrageenanlösung von innen und von außen bildet sich momentan ein sehr stabiler Biokatalysatorfaden aus.

Der erhaltene Biokatalysatorfaden wird für weitere 30 Minuten in der KCl-Lösung belassen. Anschließend wird der Katalysator mit Leitungswasser gewaschen und dabei fünf Minuten mit einer 6 Watt UV-Lampe bestrahlt. Dieser Katalysatorfaden wird nun wie in Beispiel 4 beschrieben zur Sektherstellung eingesetzt. Nach 14 Tagen ist die Zweitgärung abgeschlossen, und der Sekt wird aus dem Gärtank abgepumpt.

Die nach allen Beispielen hergestellten Biokatalysatoren verhindern wegen ihrer zellenfreien mikroporösen Schutzschicht nicht nur ein Auswachsen der Zellen aus dem Kern sondern auch ein Einwachsen von Fremdzellen. Die Kontaminationsgefahr durch Fremdzellen ist daher vermindert. Es ist somit möglich, unter verringerten Anforderungen an die Sterilität zu arbeiten. Ferner können die Biokatalysatoren getrocknet und in geeigneter Form gelagert werden. Beim späteren Aufquellen entsteht ein geringerer Wassergehalt als vor dem Trocknen, so daß die relative Zellbeladung dadurch etwas erhöht wird.

Das erfindungsgemäße Verfahren ermöglicht erstmalig die praktikable Verwendung von Carrageenan zur Immobilisierung von Zellen bei Raumtemperatur. Zellen, die nicht in einem sehr aufwendigen, mehrstufigen Verfahren extra gereinigt worden sind, weisen an sich nicht störende Verunreinigungen durch $NH_4$-, Ca-, K-Ionen usw. auf. Diese geringfügigen Ionenkonzentrationen verursachen ein Aushärten des Carrageenans auch bei höheren Temperaturen, so daß bisher die Carrageenanzugabe bei relativ hohen Temperaturen (beispielsweise 60 °C) erfolgen mußte, um nicht eine vorzeitige Aushärtung des Carrageenans zu erhalten. Da zur Bildung der erfindungsgemäßen Schutzschicht um den immobilisierten Zellkern das Carrageenan praktisch nicht mehr mit den Zellen in Berührung kommt, bleibt es auch bei niedrigeren Temperaturen flüssig und verarbeitbar und kann kontrolliert vernetzt werden.

### Patentansprüche

1. Verfahren zur Herstellung eines Biokatalysators mit immobilisierten Zellen, bei dem die Zellen in eine durch ein Vernetzungsmittel zum Gel werdende, aus einer wäßrigen Phase gebildeten Flüssigkeit eingebracht werden und anschließend durch Vernetzung der Biokatalysator mit oder ohne einen mechanischen Träger gebildet wird, dadurch gekennzeichnet, daß der die Zellen enthaltende Kern von einer gelbildenden Flüssigkeit umgeben wird und daß die Vernetzung des Gels unter Einwirkung eines aus dem Kern diffundierenden Vernetzungsmittels an dem Kern erfolgt, so daß eine von den Zellen freie Schutzschicht aus dem vernetzten, keine Durchlässigkeit für die Zellen aufweisenden Gel gebildet wird, die den die Zellen enthaltenden Kern umschließt.

2. Verfahren nach Anspruch 1, dadurch gekennzeichnet, daß für die Schutzschicht ein ionotropes Gel verwendet wird.

3. Verfahren nach Anspruch 1 oder 2, dadurch gekennzeichnet, daß der die Zellen enthaltende Kern durch Vernetzung einer gelbildenden Flüssigkeit gebildet wird, in der die Zellen suspendiert wurden.

4. Verfahren nach Anspruch 1 oder 2, dadurch gekennzeichnet, daß die Schutzschicht um einen die freien Zellen enthaltenden flüssigen Kern gebildet wird.

5. Verfahren nach Anspruch 3, dadurch gekennzeichnet, daß die Zellen der gelbildenden Flüssigkeit zugegeben werden und diese in das Vernetzungsmittel geleitet wird und daß anschließend der so gebildete Kern in eine gelbildende Flüssigkeit gegeben wird, die mit dem an der Oberfläche des Kerns haftenden Vernetzungsmittel das die Schutzschicht bildende von den Zellen freie Gel bildet.

6. Verfahren nach einem der Ansprüche 1 bis 5, dadurch gekennzeichnet, daß eine zusätzliche Vernetzung der Schutzschicht dadurch vorgenommen wird, daß der Kern von der die Schutzschicht bildenden gelbildenden Flüssigkeit umgeben wird und anschließend in ein Vernetzungsbad gelangt.

7. Verfahren nach Anspruch 6, dadurch gekennzeichnet, daß das Umgeben der die Zellen enthaltenden Flüssigkeit des Kerns mit der gelbildenden Flüssigkeit der Schutzschicht mit einer zentralen ersten Düsenöffnung und einer diese erste Düsenöffnung umgebenden ringförmigen zweiten Düsenöffnung vorgenommen wird.

8. Verfahren nach einem der Ansprüche 1 bis 7, dadurch gekennzeichnet, daß die eingeschlossenen Zellen des Kerns Hefezellen zur Durchführung einer Sektgärung sind.

9. Verfahren nach einem der Ansprüche 1 bis 8, dadurch gekennzeichnet, daß in den Kern lediglich eine Vorkultur von Zellen eingebracht wird und daß der mit der Vorkultur erstellte Katalysator in eine für die Züchtung der Zellen geeignete Umgebung eingebracht wird.

10. Biokatalysator mit immobilisierten Zellen, bei dem die Zellen in eine durch eine wäßrige Phase gebildete Flüssigkeit eingebracht sind und dessen Oberfläche durch eine Schutzschicht aus einem vernetzten, den die Zellen enthaltenden Kern mit oder ohne einen mechanischen Träger umschließenden Gel gebildet ist, dadurch gekennzeichnet, daß die Schutzschicht frei von den Zellen und mit einem aus dem Kern diffundierten Vernetzungsmittel gebildet ist.

11. Biokatalysator nach Anspruch 10, dadurch gekennzeichnet, daß die Schutzschicht durch ein ionotropes Gel gebildet ist.

12. Biokatalysator nach Anspruch 10 oder 11, dadurch gekennzeichnet, daß der Kern durch ein vernetztes Gel gebildet ist.

13. Biokatalysator nach Anspruch 12, dadurch gekennzeichnet, daß das vernetzte Gel des Kerns mit demselben Vernetzungsmittel vernetzt ist wie das Gel der Schutzschicht.

14. Biokatalysator nach Anspruch 10 oder 11, dadurch gekennzeichnet, daß der Kern durch eine unvernetzte Flüssigkeit gebildet ist.

15. Biokatalysator nach Anspruch 14, dadurch gekennzeichnet, daß der unvernetzten Flüssigkeit Verdickungsmittel zugesetzt sind.

16. Biokatalysator nach einem der Ansprüche 10 bis 15, dadurch gekennzeichnet, daß der Kern an einem mechanisch stabilen Träger verankert ist.

17. Biokatalysator nach einem der Ansprüche 10 bis 16, dadurch gekennzeichnet, daß der Kern lediglich eine Vorkultur von Zellen enthält.

18. Verfahren zur Herstellung eines Biokatalysators mit immobilisierten Zellen, bei dem die Zellen in eine durch ein Vernetzungsmittel zum Gel werdende, aus einer wäßrigen Phase gebildeten Flüssigkeit eingebracht werden und anschließend der Biokatalysator mit oder ohne einen mechanischen Träger gebildet wird, dadurch gekennzeichnet, daß die die Zellen enthaltende Flüssigkeit von der gelbildenden Flüssigkeit umgeben wird und anschließend vor einer Vermischung der die Zellen enthaltenden Flüssigkeit mit der gelbildenden Flüssigkeit in das Vernetzungsmittel gelangt, wodurch eine keine Durchlässigkeit für die Zellen aufweisende Schutzschicht durch das vernetzte Gel gebildet wird.

19. Verfahren nach Anspruch 18, dadurch gekennzeichnet, daß der die Zellen enthaltenden Flüssigkeit Vernetzungsmittel für die die Schutzschicht bildende gelbildende Flüssigkeit zugegeben wird.

20. Verfahren nach Anspruch 18 oder 19, dadurch gekennzeichnet, daß die die Zellen enthaltende Flüssigkeit mit dem Vernetzungsmittel für die gelbildende Flüssigkeit der Schutzschicht ebenfalls vernetzt.

**21.** Verfahren nach einem der Ansprüche 18 bis 20, dadurch gekennzeichnet, daß das Umgeben der die Zellen enthaltenden Flüssigkeit des Kerns mit der gelbildenden Flüssigkeit der Schutzschicht mit einer Düsenanordnung aus einer zentralen ersten Düsenöffnung und einer diese erste Düsenöffnung umgebenden ringförmigen, zweiten Düsenöffnung vorgenommen wird.

**22.** Verfahren nach Anspruch 21, dadurch gekennzeichnet, daß die gelbildende Flüssigkeit durch die ringförmige zweite Düsenöffnung mit einer höheren Transportgeschwindigkeit transportiert wird als die die Zellen enthaltende Flüssigkeit durch die zentrale erste Düsenöffnung.

**23.** Verfahren nach einem der Ansprüche 18 bis 22, dadurch gekennzeichnet, daß die eingeschlossenen Zellen des Kerns Hefezellen zur Durchführung einer Sektgärung sind.

**24.** Verfahren nach einem der Ansprüche 18 bis 23, dadurch gekennzeichnet, daß in den Kern lediglich eine Vorkultur von Zellen eingebracht wird und daß der mit der Vorkultur erstellte Katalysator in eine für die Züchtung der Zellen geeignete Umgebung eingebracht wird.

**Claims**

**1.** Process for preparing a biocatalyst having immobilized cells, in which the cells are introduced into a liquid which is gelled by a crosslinking agent and is formed from an aqueous phase, and the biocatalyst is then formed with or without a mechanical carrier by crosslinking, characterized in that the core containing the cells is surrounded by a gel-forming liquid, and in that the gel is crosslinked at the core under the action of a crosslinking agent diffusing out of the core, so that a protective layer free of the cells is formed from the crosslinked gel, which has no permeability to the cells, and this protective layer encloses the core containing the cells.

**2.** Process according to Claim 1, characterized in that an ionotropic gel is used for the protective layer.

**3.** Process according to Claim 1 or 2, characterized in that the core containing the cells is formed by crosslinking a gel-forming liquid in which the cells have been suspended.

**4.** Process according to Claim 1 or 2, characterized in that the protective layer is formed around a liquid core containing the free cells.

**5.** Process according to Claim 3, characterized in that the cells are added to the gel-forming liquid and the latter is fed into the crosslinking agent, and in that the core thus formed is then added to a gel-forming liquid which, with the crosslinking agent adhering to the surface of the core, forms the gel which forms the protective layer and is free of the cells.

**6.** Process according to one of Claims 1 to 5, characterized in that an additional crosslinking of the protective layer is carried out in that the core is surrounded by the gel-forming liquid forming the protective layer and is then passed into a crosslinking bath.

**7.** Process according to Claim 6, characterized in that the liquid of the core, containing the cells, is surrounded by the gel-forming liquid of the protective layer by means of a first, central nozzle opening and a second, annular nozzle opening surrounding this first nozzle opening.

**8.** Process according to one of Claims 1 to 7, characterized in that the enclosed cells of the core are yeast cells for performing a champagne fermentation.

**9.** Process according to one of Claims 1 to 8, characterized in that only a preliminary culture of cells is introduced into the core, and in that the catalyst produced with the preliminary culture is introduced into an environment suitable for culturing the cells.

**10.** Biocatalyst having immobilized cells, in which the cells are introduced into a liquid formed by an aqueous phase and the surface of this biocatalyst being formed by a protective layer comprising a crosslinked gel surrounding the core containing the cells, with or without a mechanical carrier, characterized in that the protective layer is free of the cells and is formed by a crosslinking agent diffusing out of the core.

**11.** Biocatalyst according to Claim 10, characterized in that the protective layer is formed by an ionotropic gel.

**12.** Biocatalyst according to Claim 10 or 11, characterized in that the core is formed by a crosslinked gel.

**13.** Biocatalyst according to Claim 12, characterized in that the crosslinked gel of the core is crosslinked with the same crosslinking agent as the gel of the protective layer.

14. Biocatalyst according to Claim 10 or 11, characterized in that the core is formed by an uncrosslinked liquid.

15. Biocatalyst according to Claim 14, characterized in that a thickening agent is added to the uncrosslinked liquid.

16. Biocatalyst according to one of Claims 10 to 15, characterized in that the core is anchored to a mechanically stable carrier.

17. Biocatalyst according to one of Claims 10 to 16, characterized in that the core contains only one preliminary culture of cells.

18. Process for preparing a biocatalyst having immobilized cells, in which the cells are introduced into a liquid which is gelled by a crosslinking agent and is formed from an aqueous phase, and the biocatalyst is then formed with or without a mechanical carrier, characterized in that the liquid containing the cells is surrounded by the gel-forming liquid and then, before mixing the liquid containing the cells with the gel-forming liquid, is passed into the crosslinking agent, as a result of which a protective layer having no permeability to the cells is formed by the crosslinked gel.

19. Process according to Claim 18, characterized in that the crosslinking agent for the gel-forming liquid forming the protective layer is added to the liquid containing the cells.

20. Process according to Claim 18 or 19, characterized in that the liquid containing the cells is also crosslinked with the crosslinking agent for the gel-forming liquid of the protective layer.

21. Process according to one of Claims 18 to 20, characterized in that the liquid, containing the cells, of the core is surrounded with the gel-forming liquid of the protective layer by means of a nozzle arrangement comprising a first, central nozzle opening and a second, annular nozzle opening surrounding this first nozzle opening.

22. Process according to Claim 21, characterized in that the gel-forming liquid is transported through the second, annular nozzle opening at a higher speed of transportation than the liquid containing the cells is transported through the first, central nozzle opening.

23. Process according to one of Claims 18 to 22, characterized in that the enclosed cells of the core are yeast cells for carrying out a champagne fermentation.

24. Process according to one of Claims 18 to 23, characterized in that only one preliminary culture of cells is introduced into the core, and in that the catalyst produced with the preliminary culture is introduced into an environment suitable for culturing the cells.

## Revendications

1. Procédé de préparation d'un biocatalyseur à cellules immobilisées, dans lequel les cellules sont introduites dans un liquide formé d'une phase aqueuse, devenant un gel sous l'effet d'un agent de réticulation et le biocatalyseur est ensuite formé par réticulation avec ou sans support mécanique, caractérisé en ce que le noyau contenant les cellules est enveloppé d'un liquide géligène et en ce que la réticulation du gel par l'action d'un agent de réticulation diffusant hors du noyau s'effectue sur le noyau, en sorte qu'une couche protectrice exempte de cellules se forme à partir du gel réticulé dépourvu de perméabilité aux cellules, laquelle couche protectrice enveloppe le noyau contenant les cellules.

2. Procédé suivant la revendication 1, caractérisé en ce que l'on utilise un gel ionotrope pour la couche protectrice.

3. Procédé suivant la revendication 1 ou 2, caractérisé en ce que le noyau contenant les cellules est formé par la réticulation d'un liquide géligène dans lequel les cellules ont été mises en suspension.

4. Procédé suivant la revendication 1 ou 2, caractérisé en ce que la couche protectrice est formée autour d'un noyau liquide contenant les cellules libres.

5. Procédé suivant la revendication 3, caractérisé en ce que l'on ajoute les cellules au liquide géligène et en ce que l'on fait passer celui-ci dans l'agent de réticulation et en ce que l'on ajoute ensuite le noyau ainsi formé à un liquide géligène qui forme le gel dépourvu de cellules constituant la couche protectrice, à l'aide de l'agent de réticulation adhérant à la surface du noyau.

**6.** Procédé suivant l'une quelconque des revendications 1 à 5, caractérisé en ce que l'on entreprend une réticulation complémentaire de la couche protectrice en enveloppant ou enrobant le noyau du liquide géligène formant la couche protectrice et en l'envoyant ensuite dans un bain de réticulation.

**7.** Procédé suivant la revendication 6, cartactérisé en ce que l'enveloppement ou l'enrobage du liquide contenant les cellules du noyau par le liquide géligène de la couche protectrice s'entreprend au moyen d'une première ouverture d'ajutage centrale et d'une seconde ouverture d'ajutage annulaire entourant la première ouverture d'ajutage.

**8.** Procédé suivant l'une quelconque des revendications 1 à 7, caractérisé en ce que les cellules emprisonnées ou enfermées du noyau sont des cellules de levure pour la réalisation d'une fermentation de vin champagnisé ou obtenu selon la méthode champenoise.

**9.** Procédé suivant l'une quelconque des revendications 1 à 8, caractérisé en ce que l'on introduit uniquement une culture préalable de cellules dans le noyau et en ce que l'on introduit le catalyseur réalisé avec la culture préalable dans un milieu convenant à la culture ou l'élevage des cellules.

**10.** Biocatalyseur à cellules immobilisées, dans lequel les cellules sont introduites par un liquide formé d'une phase aqueuse et dont la surface est formée par une couche protectrice en un gel réticulé, enveloppant ou enrobant le noyau contenant les cellules avec ou sans support mécanique, caractérisé en ce que la couche protectrice est formée de manière à être exempte des cellules et à l'aide d'un agent de réticulation diffusant hors du noyau.

**11.** Biocatalyseur suivant la revendication 10, caractérisé en ce que la couche protectrice est formée par un gel ionotrope.

**12.** Biocatalyseur suivant la revendication 10 ou 11, caractérisé en ce que le noyau est formé par un gel réticulé.

**13.** Biocatalyseur suivant la revendication 12, caractérisé en ce que le gel réticulé du noyau est réticulé avec le même agent de réticulation que le gel de la couche protectrice.

**14.** Biocatalyseur suivant la revendication 10 ou 11, caractérisé en ce que le noyau est formé par un liquide non réticulé.

**15.** Biocatalyseur suivant la revendication 14, caractérisé en ce que des agents d'épaississement ou épaississants sont ajoutés au liquide non réticulé.

**16.** Biocatalyseur suivant l'une quelconque des revendications 10 à 15, caractérisé en ce que le noyau est ancré à un support mécaniquement stable.

**17.** Biocatalyseur suivant l'une quelconque des revendications 10 à 16, caractérisé en ce que le noyau ne contient uniquement qu'une culture préalable de cellules.

**18.** Procédé de préparation d'un biocatalyseur avec des cellules immobilisées, dans lequel les cellules sont introduites dans un liquide constitué d'une phase aqueuse et se transformant en gel sous l'effet d'un agent de réticulation et on forme ensuite le biocatalyseur avec ou sans support mécanique, caractérisé en ce que le liquide contenant les cellules est enveloppé ou enrobé du liquide géligène et parvient ensuite dans l'agent de réticulation, avant un mélange du liquide contenant les cellules avec le liquide géligène, en sorte que se forme une couche protectrice dépourvue de perméabilité aux cellules à l'aide du gel réticulé.

**19.** Procédé selon la revendication 18, caractérisé en ce que l'on ajoute l'agent de réticulation pour le liquide géligène formant la couche protectrice au liquide contenant les cellules.

**20.** Procédé suivant la revendication 18 ou 19, caractérisé en ce que l'on réticule également le liquide contenant les cellules avec l'agent de réticulation pour le liquide géligène de la couche protectrice.

**21.** Procédé selon l'une quelconque des revendications 18 à 20, caractérisé en ce que l'enveloppement ou l'enrobage du liquide du noyau contenant les cellules s'entreprend avec le liquide géligène de la couche protectrice à l'aide d'un agencement d'ajutage constitué d'une première ouverture d'ajutage centrale et d'une seconde ouverture d'ajutage annulaire, entourant cette première ouverture d'ajutage.

**22.** Procédé suivant la revendication 21, caractérisé en ce que le liquide géligène est transporté à travers la seconde ouverture d'ajutage annulaire à une vitesse de transport supérieure à celle du liquide contenant les cellules à travers la première ouverture d'ajutage centrale.

23. Procédé suivant l'une quelconque des revendications 18 à 22, caractérisé en ce que les cellules enfermées ou emprisonnées du noyau sont des cellules de levure pour la réalisation d'une fermentation de vin champagnisé ou obtenu selon la méthode champenoise.

24. Procédé suivant l'une quelconque des revendications 18 à 23, caractérisé en ce que l'on introduit dans le noyau uniquement une culture préalable de cellules et en ce que l'on introduit le catalyseur réalisé avec la culture préalable dans un environnement convenant à la culture ou l'élevage des cellules.

Fig.1

# F i g.2

# Fig. 3

06423  25KV    50U

Fig.4

# F i g.5

Fig.6

14